(19) Europäisches Patentamt
European Patent Office
Office européen des brevets

(11) Numéro de publication: **0 117 797**
**A1**

(12) # DEMANDE DE BREVET EUROPEEN

(21) Numéro de dépôt: **84400265.9**

(22) Date de dépôt: **08.02.84**

(51) Int. Cl.[3]: **C 07 C 127/15,** C 07 C 149/437, C 07 D 209/20, C 07 D 207/16, A 61 K 31/17, A 61 K 31/195, A 61 K 31/395

(30) Priorité: **08.02.83 FR 8301937**

(43) Date de publication de la demande: **05.09.84**
**Bulletin 84/36**

(84) Etats contractants désignés: **AT BE CH DE FR GB IT LI LU NL SE**

(71) Demandeur: **CENTRE NATIONAL DE LA RECHERCHE SCIENTIFIQUE (C.N.R.S.), 15, Quai Anatole France, F-75007 Paris (FR)**

(72) Inventeur: **Rodriguez, Marc, 3 rue du Bayle, F-34000 Montpellier (FR)**
Inventeur: **Martinez, Jean-Alphonse-Joseph, Rue des Cévennes, F-34720 Caux (FR)**
Inventeur: **Imbach, Jean Louis, 1108, rue des Sorbes, F-34000 Montpellier (FR)**

(74) Mandataire: **Corre, Jacques Denis Paul et al, Cabinet Regimbeau 26, Avenue Kléber, F-75116 Paris (FR)**

(54) **Nouvelles nitrosourées, leur procédé de préparation et leur application en thérapeutique.**

(57) La présente invention concerne de nouvelles nitrosourées, et leur préparation et leur application en thérapeutique.

Il s'agit de composés de formule

$$Y\,[\;N(R)\text{-}A\text{-}\underset{\substack{\|\\ O}}{C}\;]\;X \qquad (I)$$

de séries L, D, DL, allo et thréo,
dans lesquels:

A représente le fragment d'un amino-acide de formule HN-(R)-A-COOH, où

R représente un atome d'hydrogène, un radical organique en particulier un radical alkyle, ou bien forme avec l'atome d'azote et A un hétérocycle;

Y représente un radical nitrosourée de formule

$$Cl\text{-}CH_2\text{-}CH_2\text{-}\underset{\substack{|\\ NO}}{N}\text{-}\overset{\substack{O\\ \|}}{C}\text{-} \quad \text{ou} \quad CH_3\text{-}\underset{\substack{|\\ NO}}{N}\text{-}\overset{\substack{O\\ \|}}{C}\text{-}\;; \qquad \text{et}$$

X représente un radical moutarde à l'azote de formule

$Cl\text{-}CH_2\text{-}CH_2\text{-}NH\text{-}$, $(Cl\text{-}CH_2\text{-}CH_2)_2N\text{-}$ ou
$(Cl\text{-}CH_2\text{-}CH_2)_2N\text{-}C_6H_4\text{-}NH\text{-}$

Application au traitement des tumeurs.

EP 0 117 797 A1

NOUVELLES NITROSOUREES, LEUR PROCEDE DE PREPARATION ET LEUR APPLICATION EN THERAPEUTIQUE.

La présente invention concerne de nouvelles nitrosourées, leur procédé de préparation et leur application en thérapeutique.

On sait que certaines halogéno-alkyl-nitrosourées, en particulier certaines 2-chloro-éthyl-nitrosourées, constituent une classe de composés particulièrement intéressants en raison de leur activité oncostatique sur des tumeurs malignes chez l'animal et chez l'homme et que ces composés sont utiles pour la chimiothérapie du cancer.

De nombreux efforts ont été mis en oeuvre pour développer de nouveaux composés de cette série pouvant présenter une plus grande efficacité antinéoplastique et/ou une toxicité plus faible.

C'est ainsi que tout récemment de nouvelles nitrosourées, dérivées d'amino-acides libres côté C-terminal ou d'amides ont été proposées. Ces dérivés ont montré une activité cytotoxique intéressante.

La présente invention concerne de nouvelles nitrosourées, dérivées d'amino-acides, qui mettent à profit le caractère poly-fonctionnel des amino-acides qui, pour les plus simples, présentent au moins deux sites réactifs : une fonction amine et une fonction acide.

Conformément à l'invention, la fonction amine permet le greffage d'une entité cytotoxique nitrosourée, tandis que la fonction acide permet le greffage d'une entité moutarde à l'azote.

L'invention concerne plus particulièrement ces composés de formule

$$Y \left[ N(R)-A-\underset{\underset{O}{\|}}{C} \right] X \qquad (I)$$

de séries L, D, DL, allo et thréo, dans lesquels :

A représente le fragment d'un amino-acide de formule $HN(R)-A-COOH$, où

R représente un atome d'hydrogène, un radical organique en particulier un radical alkyle, ou bien forme avec l'atome d'azote et A un hétérocycle ;

Y représente un radical nitrosourée de formule

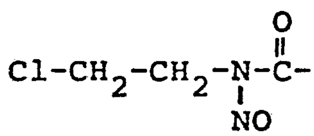 ou 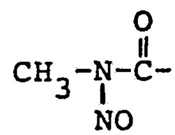 ; et

X représente un radical moutardé à l'azote de formule

$Cl-CH_2-CH_2-NH-$, $(Cl-CH_2-CH_2)_2N-$ ou $(Cl-CH_2-CH_2)_2N-C_6H_4-NH-$

Ces composés portent ainsi deux entités cytotoxiques reconnues, à savoir au moins une nitrosourée Y sur le côté N-terminal de l'amino-acide et au moins une moutarde à l'azote X sur le côté C-terminal. Dans le cas où l'acide aminé de base comporte deux fonctions acides (acide aspartique par exemple), le composé possède alors une nitrosourée et deux entités moutardes à l'azote. Dans le cas où l'acide aminé de base comporte deux fonctions amines (lysine par exemple), le composé possède alors deux entités nitrosourées et une entité moutarde à l'azote.

Il s'agit par conséquent de composés polyfonctionnels qui se différentient fondamentalement des composés monofonctionnels connus comportant une entité nitrosourée greffée sur un amino-acide.

Un premier groupe de composés selon l'invention correspond à la formule

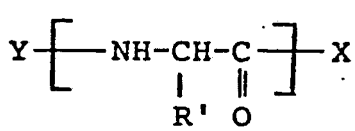 (Ia)

dans lesquels :

R' est choisi parmi les radicaux $-H, -CH_3, -CH_2OH$, $-CH(CH_3)-OH$, $-CH(CH_3)_2$, $-CH_2-CH_3$, $-CH_2-CH(CH_3)_2$,

$-CH(CH_3)-CH_2-CH_3$, 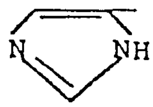 , $-CH_2-SH$, $-CH_2-CH_2-S-CH_3$,

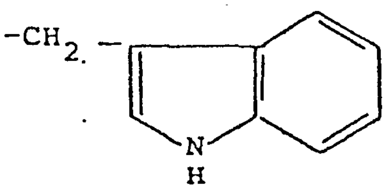 , 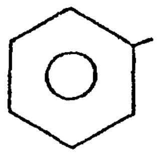 , 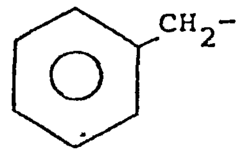 , 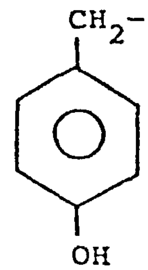 ,

$-CH_2-CO-NH_2$, $-CH_2-CH_2-CONH_2$, $-(CH_2)_nCO-X$, $-(CH_2)_n-NH-Y$, $-(CH_2)_3-NH-C(=NH)-NH-Y$, $-CH_2-CH_2SH$, $-CH_2-CH_2-OH$, $-(CH_2)_2CH-OH-CH_2-NH-Y$

n représente un nombre entier de 1 à 7 ; et X et Y ont la même signification que ci-dessus.

Ce premier groupe comprend donc essentiellement les acides aminés naturels et leurs dérivés.

Un deuxième groupe de composés selon l'invention correspond à la formule

$$Y-\left[NH-(CH_2)_n-\underset{\underset{O}{\|}}{C}\right]-X \qquad (Ib)$$

dans lesquels :
n représente un nombre entier de 1 à 7 ; et
X et Y ont la même signification que ci-dessus.

Ce deuxième groupe comprend donc des amino-acides non naturels puisqu'ils ne peuvent être obtenus que par synthèse.

Un troisième groupe de composés selon l'invention correspond à la formule

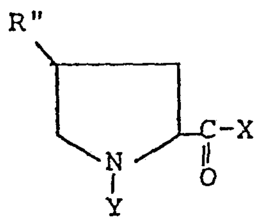 (Ic) ou 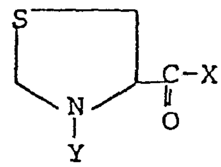 (Id)

dans lesquels

$R''$ représente un radical organique en particulier un radical alkyle ; et

X et Y ont la même signification que ci-dessus.

Parmi les composés préférés de l'invention, il faut citer ceux pour lesquels A dans la formule I représente le fragment d'un amino-acide choisi parmi Ala, β Ala, Asp, Asn, Gly, GABA, Ile, Leu, Lys, Met, Phe, Pro, Trp et Tyr.

Le procédé de préparation des composés de formule I peut être représenté par le schéma suivant :

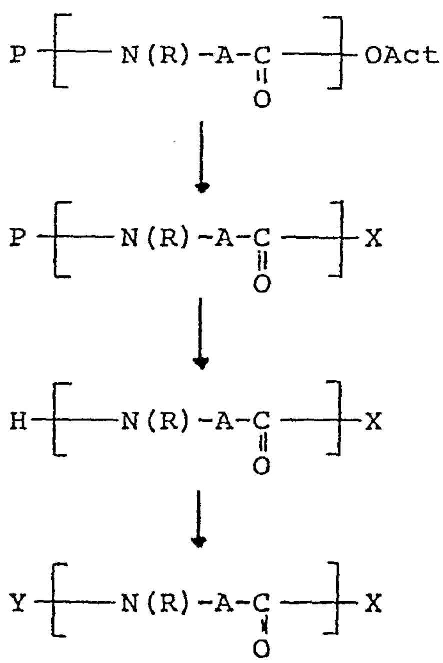

Le procédé de l'invention comporte les étapes suivantes :

On fait tout d'abord réagir un composé de formule

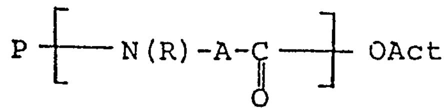 (II)

où A a la signification ci-dessus, P représente un groupement protecteur et Act un groupement activant, sur le chlorhydrate de l'amine de formule

HX (III)

où X a la signification ci-dessus, pour donner un composé de formule

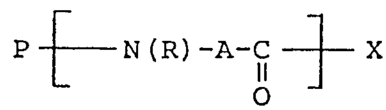 (IV)

On hydrogène ensuite le composé de formule IV pour donner un composé de formule

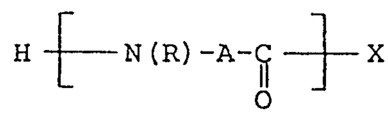 (V)

On fait ensuite réagir le composé de formule V avec un nitrosocarbamate de formule

Y—OAct (VI)

où Y et Act ont la signification ci-dessus.

Dans le procédé ci-dessus, on a utilisé comme entités nitrosourées la 2-chloroéthyl-nitrosourée et la méthyl-nitrosourée et comme entités moutarde à l'azote, la 2-chloroéthylamine, la bis-(2-chloroéthyl)

amine et la N-N-bis(2-chloroéthyl) phénylène diamine.

Le groupement N-protecteur P peut être indifféremment un groupement benzyloxy-carbonyle (Z) ou tert-butyloxy-carbonyle (Boc). Toutefois, dans le cas d'amino-acides contenant du soufre, on préfère utiliser le groupement Boc.

L'activationest de préférence réalisée par un ester activé. Act représente avantageusement un radical paranitrophénol ou N-hydroxysuccinimide.

L'introduction de la 2-chloroéthyl-nitrosourée et de la méthyl-nitrosourée s'effectue avantageusement à partir de N(2-chloroéthyl)-N-nitrosocarbamates activés ou respectivement de N-méthyl-N-nitrosocarbamate activés.

Dans le procédé ci-dessus, la réaction du composé de formule II avec le composé de formule III est effectuée en présence d'un solvant organique, par exemple le diméthylformamide (DMF). On distille ensuite le solvant et on reprend le résidu dans l'acétate d'éthyle puis on sèche et concentre la phase organique obtenue et on recristallise le résidu obtenu.

L'hydrogénation du composé de formule IV est réalisée dans un solvant organique, par exemple dans de l'éthane, en présence de palladium sur charbon, sous pression normale et à température ambiante. On élimine ensuite le catalyseur par filtration, puis on concentre à sec et sèche le filtrat obtenu.

Le composé de formule V ainsi obtenu est dissout dans un solvant organique et on y ajoute le nitrosocarbamate de formule VI. On abandonne alorsle mélange réactionnel à la température ambiante puis on le concentre sous pression réduite. On chromatographie alors le résidu obtenu puis on recristallise le composé pur de formule I ainsi obtenu.

La préparation des nitrosourées de l'invention sera maintenant décrite avec plus de détails à l'aide des exemples non limitatifs suivants :

Exemple 1

Mode opératoire général pour l'obtention d'un 2-chloro-éthylamine d'amino-acide N-protégé

A une solution refroidie à 0°C de 2,32 g (0,02 mole) de chlorhydrate de 2-chloro-éthylamine et de 3,45 ml (0,02 mole) de diisopropyléthylamine dans 30 ml de DMF, on ajoute 0,01 mole d'ester activé (du paranitrophénol ou du N-hydroxysuccinimide) d'un amino acide N-protégé. Le mélange réactionnel est agité pendant une nuit à température ambiante.

Le solvant est alors distillé sous pression réduite. Le résidu est dissout dans 200 ml d'acétate d'éthyle. Cette solution est lavée par 2 x 100 ml d'une solution aqueuse à 10 % d'acide citrique, 2 x 100 ml d'eau, 2 x 100 ml d'une solution aqueuse saturée de bicarbonate de sodium, 2 x 100 ml d'eau. La phase organique est séchée sur sulfate de sodium et concentrée sous pression réduite.

Le résidu obtenu est, selon le cas, directement recristallisé, ou bien recristallisé après chromatographie sur colonne de silice.

En suivant ce mode opératoire général, on prépare les composés des exemples 1.1 à 1.9 ci-dessous :

Exemple 1.1.

N-t-Butyloxycarbonyl β alanine 2-chloro-1-éthylamide

A partir de l'ester de N-hydroxysuccinimide de la N-t-Butyloxycarbonyl β alanine.

Recristallisation dans un mélange de dichlorométhane et d'éther de pétrole (1/1)

Rendement 65 %

F = 25 - 87°C

Analyse : $C_{10}H_{19}N_2O_3Cl$ (C,H,N)

CCM : Rf = 0,40 (Acétate d'éthyle)

Exemple 1.2

2-chloro-éthylamide de l'acide N-carbobenzoxy 4-amino-2-butanoïque

A partir de l'ester de N-hydroxysuccinimide de l'acide N-carbobenzoxy-4-amino butanoïque.

Recristallisation dans un mélange d'acétate d'éthyle et d'éther de pétrole (1/1)

Rendement 63 %

F = 97 - 99°C

Analyse : $C_{14}H_{19}N_2O_3Cl$ (C,H,N)

CCM : Rf = 0,35 (Acétate d'éthyle)

Exemple 1.3

N-t-butyloxycarbonyl-L-alanine-2-chloro-3-éthylamide

A partir de l'ester paranitrophénylique de la N-t-Butyloxycarbonyl-L-alanine.

Le résidu est chromatographié sur colonne de silice. Eluant dichlorométhane/éther 1/1.

Recristallisation dans un mélange d'éther et d'éther de pétrole (1/3)

Rendement 76 %

F = 101-103°C

Analyse : $C_{10}H_{19}N_2O_3Cl$ (C,H,N)

CCM : Rf = 0,68 (Dichlorométhane/Ether-2/1)

$(\alpha)^{20}_{D}$ = -8,1° (C=1, DMF)

Exemple 1.4

N-carbobenzoxy-L-isoleucine-2-chloro-4-éthylamide

A partir de l'ester de N-hydroxysuccinimide de la N-carbobenzoxy-L-isoleucine.

Recristallisation dans l'éthanol.

Rendement 78 %

F = 175 - 177°C

Analyse : $C_{16}H_{23}N_2O_3Cl_2$ (C,H,N)

CCM : Rf = 0,68 (acétate d'éthyle/dichloro-méthane-1/1)

$(\alpha)^{20}_{D}$ = +3,5° (C = 1 ; DMF)

Exemple 1.5

N-carbobenzoxyl-L-leucine-2-chloro-3-éthylamide

A partir de l'ester N-hydroxysuccinimide de la N-carbobenzoxy-L-leucine.

Recristallisation dans un mélange d'acétate d'éthyle et d'éther de pétrole (1/5)

Rendement 91 %

F = 101 - 103°C

Analyse : $C_{16}H_{23}N_2O_3Cl$ (C,H,N)

CCM : Rf = 0,65 (acétate d'éthyle/dichloro-méthane-1/1)

$(\alpha)^{20}_{D}$ = -8,8° (c=2,5 DMF)

Exemple 1.6

N-t-Butyloxycarbonyl-L-méthionine-2-chloro-6-éthylamide

A partir de l'ester de N-hydroxysuccinimide de la N-t-Butyloxycarbonyl-L-méthionine.

Obtenu sous forme d'huile et utilisé sans purification supplémentaire pour l'étape suivante.

Exemple 1.7

N-tertio-butyloxycarbonyl sarcosine (2-chloro-éthyl)-7-amide

A partir de l'ester de N-hydroxysuccinimide de la N-tertiobutyloxycarbonyl sarcosine.

Recristallisation dans un mélange d'acétate d'éthyle et d'hexane (1/1)

Rendement 80 %

F = 119°C

Analyse = $C_{10}H_{19}N_2O_3Cl$ (C,H,N)

CCM : Rf : 0,41 (Acétate d'Ethyle)

Exemple 1.8

$N^{\alpha}_{1}$ $N^{\varepsilon}$ -Dicarbobenzoxy-L-Lysine-2-chloro-8-éthylamide

A partir de l'ester de N-Hydroxysuccinimide de la $N^{\alpha}$ , $N^{\varepsilon}$ -Dicarbobenzoxy-L-lysine.

Recristallisation dans un mélange d'Acétate et d'Hexane (1/1).

Rendement 88 %

F = 123-125°C

Analyse = $C_{24}H_{30}N_3O_5Cl$ (C,H,N)

CCM : Rf = 0,52 (Acétate d'Ethyle)

$(\alpha)^{20}_{D}$ = + 4,2 (c=1 DMF)

Exemple 1.9

α,β-dichloro-2 éthylamide de l'acide N-carbobenzoxy-L-9-aspartique

A une solution à 0°C de 5,34 g (0,02 mole) d'acide N-carbobenzoxy-L-aspartique dans 70 ml de DMF, on ajoute successivement 9,28 g (0,08 mole) de chlorhydrate de 2-chloro-éthylamine, 13,8 ml (0,08 mole) de diisopropyléthylamine, et 8,24 g (0,04 mole) de N,N' dicyclohexylcarbodiimide.

Aubout de 20 h à température ambiante, on filtre la N,N'-dicyclohexylurée formée, concentre le filtrat sous pression réduite, reprend le résidu par 200 ml d'acétate d'éthyle la solution obtenue est traitée comme décrit dans la méthode générale.

Les cristaux blancs obtenus sont recristallisés dans de l'alcool isopropylique.

Rendement 2,4 g soit 31 %
F = 174 - 176°C
Analyse : $C_{16}H_{21}N_3O_4Cl_2$ (C,H,N)
CCM : Rf = 0,43 (dichlorométhane/ méthanol 94/6)
$(\alpha)_D^{20}$ = +2,6 (C = 1,DMF)

Exemple 2

Modes opératoires généraux pour l'obtention des 2-chloro-éthyl nitrosocarbamoyl amino acides amides (substitués ou non).

Procédé A

(0,01 mole) de N-carbobenzoxy amino acide amide est hydrogénée en solution dans 100 ml d'éthane en présence de palladium sur charbon à 10 % et d'un équivalent d'une solution aqueuse à 10 % d'acide chlorhydrique, sous pression normale et à température ambiante.

Lorsqu'une CCM révèle l'absence de composé de départ, le catalyseur est éliminé par filtration sur papier, le filtrat est concentré à sec sous pression réduite. Le résidu est trituré dans de l'éther anhydre jusqu'à obtention d'une poudre qui est collectée, lavée à l'éther et séchée au dessicateur.

Le dérivé N-déprotégé est alors dissout dans 20 ml de DMF. Un équivalent (0,01 mole ; 172 ml) de diéthylamine DIEA est ajouté à froid, puis 0,012 mole (3,98 g) de N-2-(chloro-éthyl)N-nitroso carbamate de 2,4,5-trichloro-phényle.

Le mélange réactionnel est abandonné pendant 3 h à température ambiante, puis concentré sous pression réduite. Le résidu est chromatographié sur colonne de silice. Le produit pur obtenu est recristallisé dans le solvant approprié.

Le rendement est calculé à partir du dérivé N-protégé.

<u>Procédé B</u>

0,01 mole de N-t-butyloxycarbonyl amino acide amide est dissoute dans 20 ml d'acide trifluoroacétique anhydre en présence de 2 % de thioanisole à température ambiante. Au bout de 40 mn, la solution est concentrée sous pression réduite ; le résidu est trituré dans de l'éther anhydre jusqu'à obtention d'une poudre.

La suite des opérations s'effectue comme dans le procédé A.

En suivant le procédé A ou le procédé B ci-dessus, on prépare les composés suivants.

Exemple 2.1

N-(2-chloro-éthyl)N-nitrosocarbamoyl β alanine (2-chloro-éthyl)-10-amide

Obtenu à partir du précurseur de l'exemple 1.1. Procédé B. Eluant de chromatographie : dichlorométhane / acétate d'éthyle (3/1). Recristallisation dans un mélange d'acétate d'éthyle et d'éther de pétrole (1/1).

Rendement: 66 %

F = 84 - 85°C (déc.)

Analyse : $C_8H_{14}N_4O_3Cl_2$ (C, H, N)

CCM : Rf = 0,58 (acétate d'éthyle)

IR : $\nu$ = 1470 $cm^{-1}$ (N-NO)

Exemple 2.2

(2-Chloro-éthyl)amide de l'acide 4-/N-(2-chloro-éthyl) N-nitrosouréido /-11-butanoïque

Obtenu à partir du précurseur de l'exemple 1.2. Procédé A Eluant de chromatographie : acétate d'éthyle. Recristallisation dans un mélange d'éther et d'hexane (1/1)

Rendement : 79 %

F = 63 - 64°C (déc.)

Analyse : $C_9H_{16}N_4O_3Cl_2$ (C,H,N)

CCM : Rf = 0,45 (acétate d'éthyle)

IR : $\nu$ = 1485 $cm^{-1}$ (N-NO)

Exemple 2.3

N-(2-chloro-éthyl) N-nitrosocarbamoyl-L-alanine (2-chloro-éthyl)-12-amide

Obtenu à partir du précurseur de l'exemple 1.3 - Procédé B
Eluant de chromatographie : dichlorométhane / acétate d'éthyle (9/1)
Recristallisation dans un mélange de dichlorométhane et d'hexane (1/1)

Rendement : 60 %

F = 112 - 114°C (déc.)

Analyse : $C_8H_{14}N_4O_3Cl_2$ (C,H,N)

CCM : Rf = 0,43 (dichlorométhane/ acétate d'éthyle - 2/1)

IR = $\nu$ = 1475 $cm^{-1}$ (N-NO)

$(\alpha)_D^{20}$ = + 43,2 (c = 1, DMF)

Exemple 2.4

N-(2-chloro-éthyl)N-nitrosocarbamoyl-L-isoleucine(2-chloro-éthyl)-13-amide

Obtenu à partir du précurseur de l'exemple 1.4 - Procédé A
Eluant de chromatographie : dichlorométhane / acétate d'éthyle (3/1)
Recristallisation dans l'éther.

Rendement : 67 %

F = 103 - 104°C (déc.)

Analyse : $C_4H_{20}N_4O_3Cl_2$ (C,H,N)

CCM : Rf = 0,58 (dichlorométhane/ acétate d'éthyle 1/1)

IR = $\nu$ = 1480 $cm^{-1}$ (N-NO)

$(\alpha)_D^{20}$ = + 37,2 (c = 1, DMF)

Exemple 2.5

N-(2-chloro-éthyl)N-nitrosocarbamoyl-L-leucine (2-chloro-éthyl)-14-amide

Obtenu à partir du précurseur de l'exemple 1.5 - Procédé A
Eluant de chromatographie : acétate d'éthyle / éther de pétrole 3/1
Recristallisation dans un mélange d'acétate d'éthyle et d'éther de pétrole (1/10)

Rendement : 71 %

F = 117 - 118°C (déc.)

Analyse : $C_{11}H_{20}N_4O_3Cl_2$ (C,H,N)

CCM = Rf = 0,52 (acétate d'éthyle / hexane 2/1)

IR = $\nu$ = 1430 $cm^{-1}$ (N-NO)

$(\alpha)_D^{20}$ = + 18,5 (c = 1, DMF)

Exemple 2.6

N-(2-chloro-éthyl) N-nitrosocarbamoyl-L-méthionine (2-chloro-éthyl)-15-amide

A partir du précurseur de l'exemple 1.6 - Procédé B
Eluant de chromatographie : éther
Recristallisation dans un mélange d'éther et d'éther de pétrole (1/1)

Rendement : 63 %

F = 53-55°C

Analyse : $C_{10}H_{18}N_4O_3SCl$ (C,H,N)

CCM : Rf = 0,64(éther)

IR = $\nu$ = 1485 $cm^{-1}$ (N-NO)

$(\alpha)_D^{20}$ = + 9,5 ( c = 1, DMF)

Exemple 2.7

N-(2-chloro-éthyl)N-nitrosocarbamoyl sarcosine (2-chloro-éthyl)-16-amide

A partir du précurseur de l'exemple 1.7 - Procédé B
Eluant de chromatographie : dichlorométhane / acétate d'éthyle (9-1)
Recristallisation dans un mélange d'éther et d'hexane (1/1)

Rendement 63 %

F = 63 - 65°C

Analyse : $C_8H_{14}N_4O_3Cl_2$ (C, H,N)

IR : ν(N-NO) = 1470 $cm^{-1}$

Rf = 0,36 (acétate d'éthyle/dichlorométhane 1/1)

Exemple 2.8

$N^{\alpha}$, $N^{\varepsilon}$ -di(2-chloro-éthyl)$N^{\alpha}$,$N^{\varepsilon}$ dimitrosocarbamoyl-L-lysine (2-chloro-éthyl)-17-amide

A partir duprécurseur de l'exemple 1.8.
Procédé A modifié, en ce sens que l'on introduit des quantités doubles de DIEA et de nitrosocarbamate "activé", puisque la lysine comporte deux fonctions amine.
Eluant de chromatographie : dichlorométhane / acétate d'éthyle (9/1)
Recristallisation dans un mélange d'acétate d'éthyle et d'éther de pétrole (1/4)

Rendement : 52 %

F = 69°C (déc.)

Analyse : $C_{14}H_{24}N_5O_7Cl_3$ (C,H,N)

CCM = Rf = 0,40 (acétate d'éthyle / hexane 1/1)

IR : $\nu$ = 1470, 1480 $cm^{-1}$ (N-NO)

$(\alpha)_D^{20}$ = + 12,3 ( c = 1, DMF)

Exemple 2.9

α, β-di/(2-chloro-éthyl)amide/ de l'acide N-(2-chloro-éthyl)N-nitrosocarbamoyl-L-18-aspartique

A partir du précurseur de l'exemple 1.9 - Procédé A
Le produit brut de la réaction est trituré dans de l'eau, séché, trituré dans de l'éther, séché. La poudre obtenue est recristallisée dans un mélange d'acétone et d'hexane (1/1).

Rendement : 70 %

F = 156-157°C (déc.)

Analyse : $C_{11}H_{18}N_5O_4Cl_3$ (C,H,N)

CCM : Rf = 0,29 (chloroforme /acétate d'éthyle 3/1)

IR : $\nu$ = 1475 $cm^{-1}$ (N-NO)

$(\alpha)_D^{20}$ = + 23,0 ( c = 1, DMF)

Les composés de l'invention présentent, comme les nitrosourées connues, une activité oncostatique, anti-néoplastique et antimitotique.

Ces composés constituent par conséquent des médicaments anti-cancéreux pour le traitement des tumeurs malignes chez l'homme et l'animal.

L'activité oncostatique de certains composés de l'invention par comparaison à d'autres nitrosourées connues a été déterminée en suivant le protocole expérimental suivant :

Des souris $F_1$, (DBA/2XC57 B 1/6) sont inoculées avec $10^5$ cellules de leucémie L 1210 par voie intrapéritonéale au jour 0 et divisées en 5 groupes. Le premier est le groupe de contrôle et les quatre autres sont traités avec les produits à tester à différentes concentrations, aux jours 1,5 et 9. Les composés sont injectés en suspension dans l'huile. La mortalité des animaux est observée et l'autopsie indique si elle est due à la leucémie ou à l'action toxique de la drogue. Les résultats, pour chaque dose de produit, sont exprimés en T/C (%) (T représente la médiane de survie du groupe traité et C la médiane de survie du groupe témoin T/C = ∞ quand 50 % des animaux sont guéris. T/C $\geqslant$ 125 % signifie qu'une drogue est active.

Ces essais ont été effectués à l'aide de composés de formule

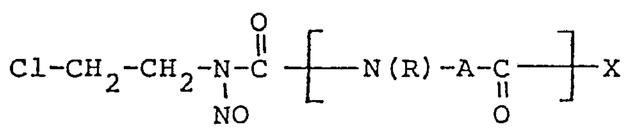

pour différents amino-acides et différentes significations du substituant X (substituant côté C terminal).

Les significations de X sont les suivantes :

1) Composés de l'invention : X = $-NH-CH_2-CH_2Cl$, $-N(CH_2-CH_2-Cl)_2$ et $-NH-C_6H_4-N(CH_2-CH_2-Cl)_2$ ,

2) Composés en dehors de l'invention : X = $-NH_2$ et $-NH-CH_3$.

Les résultats sont exprimés dans le tableau ci-après.

TABLEAU DES RESULTATS D'ACTIVITE CONTRE LA L 1210 (i.p.)

| N° | ACIDE AMINE | SUBSTITUANT X COTE C TERMINAL | $DL_{50}$ mg/kg | T/C max | M.E.D.I. mg/kg | Dose optimale mg/kg | $I = \frac{DL_{50}}{D.Opt.}$ |
|---|---|---|---|---|---|---|---|
| 1 | Ala | $-NH\ CH_2CH_2Cl$ | 35 | ∞ | 10-30 | 20 | 1,75 |
| 2 | βAla | -id- | 100 | ∞ | 20-50 | 35 | 2,86 |
| 3 | Asp[x] | -id- | 130 | ∞ | 25-50 | 37 | 3,50 |
| 4 | Asn | -id- | 35 | ∞ | 10-30 | 20 | 1,75 |
| 5 | Gly | -id- | 25 | ∞ | 5-20 | 12 | 2,00 |
| 6 | Gly | $-N(CH_2CH_2Cl)_2$ | 110 | ∞ | 25-60 | 40 | 2,70 |
| 7 | GABA | $-NH\ CH_2CH_2Cl$ | 125 | ∞ | 20-50 | 35 | 3,57 |
| 8 | Ile, | -id- | 130 | ∞ | 30-75 | 50 | 2,60 |
| 9 | Leu | -id- | 64 | ∞ | 15-50 | 35 | 1,80 |
| 10 | Leu | $-NH_2$ | 40 | | Maximum T/C = 211 % à 15 mg/kg | | |
| 11 | Leu | $-NH-CH_3$ | 33 | ∞ | 10-20 | 15 | 2,20 |
| 12 | Lys[x] | $-NH\ CH_2CH_2Cl$ | 135 | ∞ | 20-40 | 30 | 4,50 |
| 13 | Met | -id- | 32 | ∞ | 10-30 | 20 | 1,60 |
| 14 | Met | $-NH_2$ | 30 | | Maximum T/C = 236 % à 20 mg/kg | | |
| 15 | Phe | $-NH-CH_2CH_2Cl$ | 80 | ∞ | 40-60 | 50 | 1,60 |

| N° | ACIDE AMINE | SUBSTITUANT X COTE C TERMINAL | $DL_{50}$ mg/kg | T/C | M.E.D.I. mg/kg | Dose optimale mg/kg | $I = \frac{DL_{50}}{D.Opt.}$ |
|---|---|---|---|---|---|---|---|
| 16 | Phe | $-NH_2$ | 55 | | Maximum | T/C = ∞ à 40 mg/kg | |
| 17 | Phe | $-NH-CH_3$ | 38 | ∞ | 15-30 | 22 | 1,70 |
| 18 | Phe | $-NH-\phi-N(CH_2CH_2Cl)_2$ | 70 | | Maximum | T/C = 173 % à 20 mg/kg | |
| 19 | Pro | $-NH\ CH_2CH_2Cl$ | 80 | | Maximum | T/C = 194 % à 35 mg/kg | |
| 20 | Trp | -id- | 65 | ∞ | 20-35 | 27 | 2,40 |
| 21 | Tyr | -id- | 40 | | Maximum | T/C = 211 % à 25 mg/kg | |

x Les dérivés de Asp et Lys comportent respectivement 2 fonctions $-NH\ CH_2CH_2Cl$ et

2 fonctions $-NH-\underset{\displaystyle \overset{\|}{O}}{C}-\underset{\displaystyle \overset{|}{NO}}{N}-CH_2CH_2Cl$

Le tableau précédent rapporte les valeurs $DL_{50}$, T/C, M.E.D.I. (Maximum efficace dose internal = plateau d'activité maximum correspondant à T/C = ∞), dose optimale et Indice thérapeutique.

Les résultats du tableau montrent que les composés 2-chloro-éthylamides de l'invention sont plus efficaces que les composés amides connues ($-NH_2$ côté C terminal).

Ainsi les composés N° 9, 13 et 15 sont beaucoup plus efficaces que les composés N° 10, 14 et 16 comme le montre le tableau ci-dessous :

Dérivés de LEU

| N° | Substituant côté C terminal | |
|---|---|---|
| 9 | $-NH\ CH_2\ CH_2\ Cl$ | MEDI entre 15 et 50 mg/kg |
| 10 | $-NH_2$ | pas de plateau d'activité maximum |

Dérivés de MET

| | | |
|---|---|---|
| 13 | $-NH\ CH_2\ CH_2\ Cl$ | MEDI entre 10 et 30 mg/kg |
| 14 | $-NH_2$ | pas de plateau d'activité maximum |

Dérivés de PHE

| | | |
|---|---|---|
| 15 | $-NH\ CH_2\ CH_2\ Cl$ | MEDI entre 40 et 60 mg/kg |
| 16 | $-NH_2$ | pas de plateau d'activité maximum |

Il faut noter également que les valeurs $DL_{50}$ montrent que les composés sont peu toxiques.

L'invention concerne également les compositions pharmaceutiques contenant comme principe actif un composé de formule I défini plus haut.

## REVENDICATIONS

1. Composés de formule

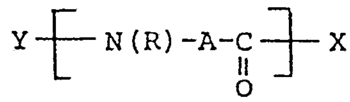 (I)

de séries L, D, DL, allo et thréo,
dans lesquels :
A représente le fragment d'un amino-acide de formule HN(R)-A-COOH, où
R représente un atome d'hydrogène, un radical organique en particulier un radical alkyle, ou bien forme avec l'atome d'azote et A un hétérocycle ;
Y représente un radical nitrosourée de formule

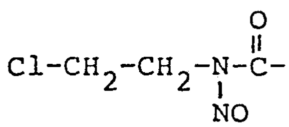 ou 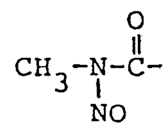 ; et

X représente un radical moutarde à l'azote de formule

$Cl-CH_2-CH_2-NH-$, $(Cl-CH_2-CH_2)_2N-$ ou $(Cl-CH_2-CH_2)_2N-C_6H_4-NH-$

2. Composés selon la revendication 1 de formule :

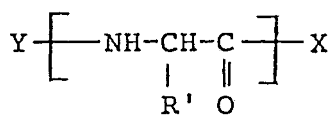 (Ia)

dans lesquels :

R' est choisi parmi les radicaux $-H$, $CH_3$, $-CH_2OH$, 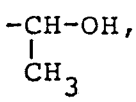, $-CH(CH_3)_2$, $-CH_2-CH_3$, $-CH_2-CH(CH_3)_2$,

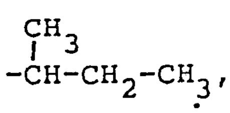, 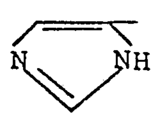, $-CH_2-SH$, $-CH_2-CH_2-S-CH_3$,

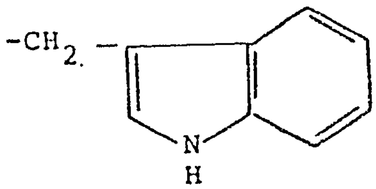 , 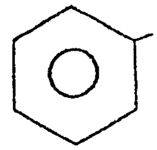 , 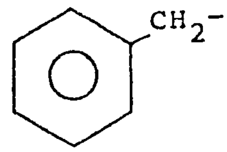 , 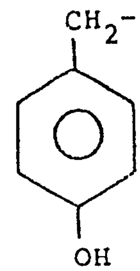 ,

$-CH_2-CO-NH_2$, $-CH_2-CH_2-CONH_2$, $-(CH_2)_nCO-X$, $-(CH_2)_n-NH-Y$, $-(CH_2)_3-NH-C(=NH)-NH-Y$, $-CH_2-CH_2SH$, $-CH_2-CH_2-OH$, $-(CH_2)_2CH-OH-CH_2-NH-$

n représente un nombre entier de 1 à 7 ; et

X et Y ont la même signification que dans la revendication 1.

3. Composés selon la revendication 1 de formule :

$$Y\left[-NH-(CH_2)_n-\underset{\underset{O}{\parallel}}{C}-\right]-X \qquad (Ib)$$

dans lesquels :

n représente un nombre entier de 1 à 7 ; et

X et Y ont la même signification que dans la revendication 1.

4. Composés selon la revendication 1 de formule :

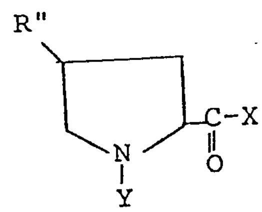 (Ic) ou 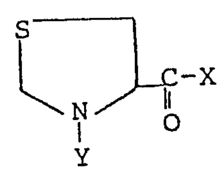 (Id)

dans lesquels

R" représente un radical organique en particulier un radical alkyle ;

X et Y ont la même signification que dans la revendication 1.

5. Composés de formule I selon la revendication 1, dans lesquels A représente le fragment d'un amino-acide choisi parmi Ala, β Ala, Asp, Asn, Gly, GABA, Ile, Leu, Lys, Met, Phe, Pro, Trp et Tyr.

6. Procédé de préparation d'un composé de formule

$$Y \left[ N(R)-A-\underset{\underset{O}{\parallel}}{C} \right] X \qquad (I)$$

dans lequel A, X et Y ont la signification donnée dans la revendication 1, caractérisé par le fait que l'on fait réagir un composé de formule

$$P \left[ N(R)-A-\underset{\underset{O}{\parallel}}{C} \right] OAct \qquad (II)$$

où A a la signification ci-dessus, P représente un groupement protecteur et Act un groupement activant, sur le chlorhydrate de l'amine de formule

$$HX \qquad (III)$$

où X a la signification ci-dessus, pour donner un composé de formule

$$P \left[ N(R)-A-\underset{\underset{O}{\parallel}}{C} \right] X \qquad (IV)$$

que l'on hydrogène le composé de formule IV pour donner un composé de formule

$$H \left[ N(R)-A-\underset{\underset{O}{\parallel}}{C} \right] X \qquad (V)$$

que l'on fait réagir le composé de formule (V) avec un nitrosocarbamate de formule

$$Y\text{-}OAct \qquad (VI)$$

où Y et Act ont la signification ci-dessus. pour donner le composé de formule I.

7. Procédé selon la revendication 6, caractérisé par le fait que P est un groupe benzyloxycarbonyle (Z) ou un groupe t-butyloxycarbonyle (Boc) et Act est un radical paranitrophénol ou N-hydroxysuccinimide.

8. Procédé selon l'une des revendications 6 et 7, caractérisé par le fait que l'on effectue la réaction du composé de formule II avec le composé de formule III en présence d'un solvant organique, que l'on distille ensuite le solvant, que l'on reprend le résidu dans l'acétate d'éthyle, qu'on sèche et concentre la phase organique obtenue, et que l'on recristallise le résidu obtenu.

9. Procédé selon la revendication 6, caractérisé par le fait que l'on hydrogène le composé de formule IV dans un solvant organique en présence de palladium sur charbon, que l'on élimine le catalyseur par filtration et que l'on concentre à sec et sèche le filtrat obtenu.

10. Procédé selon l'une des revendications 6 et 9, caractérisé par le fait que l'on dissout le composé de formule V dans un solvant organique et qu'on y ajoute le nitrosocarbamate de formule VI, que l'on concentre le mélange réactionnel sous pression réduite après abandon à la température ambiante, que l'on chromatographie le résidu et que l'on recristallise le composé pur de formule I ainsi obtenu.

11. A titre de médicament convenant notamment au traitement des tumeurs, un composé selon l'une des revendications 1 à 5.

12. Composition pharmaceutique contenant comme principe actif un composé selon l'une des revendications 1 à 5.

0117797

Office européen des brevets

# RAPPORT DE RECHERCHE EUROPEENNE

Numéro de la demande

EP 84 40 0265

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | EUROPEAN JOURNAL OF MEDICINAL CHEMISTRY, vol. 17, no. 4, 1982, pages 383-387 M. RODRIGUEZ et al.: "Synthèse et propriétés oncostatiques de dérivés peptidiques liés de facon covalente à des entités cytotoxiques nitrosourées et moutardes à l'azote" * Pages 383-387 * | 1,11, 12 | C 07 C 127/15<br>C 07 C 149/437<br>C 07 D 209/20<br>C 07 D 207/16<br>A 61 K 31/17<br>A 61 K 31/195<br>A 61 K 31/395 |
| | --- | | |
| Y | GB-A-2 091 261 (TETSUO SUAMI)<br>* Revendications * | 1,11, 12 | |
| | --- | | |
| P,Y | WO-A-8 300 860 (STIFTUNG DEUTSCHES KREBSFORSCHUNGSZENTRUM)<br>* Revendications * | 1,11, 12 | |
| | ----- | | DOMAINES TECHNIQUES RECHERCHES (Int. Cl. 3) |
| | | | C 07 C 127/00<br>C 07 C 149/00<br>C 07 D 209/00<br>C 07 D 207/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 08-05-1984 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant